**Europäisches Patentamt**

**European Patent Office**

**Office européen des brevets**

(19)

(11) Veröffentlichungsnummer: **0 058 364**
**B1**

(12)

# EUROPÄISCHE PATENTSCHRIFT

(45) Veroffentlichungstag der Patentschrift:
08.05.85

(21) Anmeldenummer. 82100877.8

(22) Anmeldetag: 08.02.82

(51) Int. Cl.⁴: **C 12 P 19/06**

(54) **Verbessertes Verfahren zur Herstellung von Xanthomonas-Biopolymeren.**

(30) Priorität: 16.02.81 DE 3105556

(43) Veröffentlichungstag der Anmeldung:
25.08.82 Patentblatt 82/34

(45) Bekanntmachung des Hinweises auf die Patenterteilung:
08.05.85 Patentblatt 85/19

(84) Benannte Vertragsstaaten:
AT BE CH DE FR GB IT LI NL SE

(56) Entgegenhaltungen:
FR - A - 2 215 465
GB - A - 1 174 322
US - A - 2 924 555
US - A - 3 284 393
US - A - 3 880 739

(73) Patentinhaber: Henkel Kommanditgesellschaft auf
Aktien, Postfach 1100 Henkelstrasse 67,
D-4000 Düsseldorf-Holthausen (DE)

(72) Erfinder: Engelskirchen, Konrad, Dr., Gonellastrasse 24,
D-4005 Meerbusch (DE)
Erfinder: Stein, Werner, Dr., Am Ginsterberg 10,
D-4000 Düsseldorf 12 (DE)
Erfinder: Bahn, Michael, Dr., Frans-Hals-Weg 19,
D-4010 Hilden (DE)
Erfinder: Schieferstein, Ludwig, Dr., Kaisersfeld 22a,
D-4200 Oberhausen (DE)
Erfinder: Schindler, Joachim, Dr., Am Eichelkamp 158,
D-4010 Hilden (DE)
Erfinder: Schmid, Rolf, Dr., Am Nettchesfeld 30,
D-4000 Düsseldorf 13 (DE)

0 058 364

## Beschreibung

Xanthangum bzw. Xanthan, auch Polysaccharid B 1459 genannt, ist ein exozelluläres Heteropolysaccharid, das von verschiedenen Xanthomonasarten, zum Beispiel Xanthomonas campestris produziert wird. Zahlreiche Veröffentlichungen befassen sich mit der Herstellung dieser hydrophilen Kolloide durch die aerobe Vermehrung und Züchtung von Bakterien der Gattung Xanthomonas in wäßrigen Nährmedien. Die grundlegenden Arbeiten sind in der US-PS 3 000 790 beschrieben. Abwandlungen des Fermentationsverfahrens sind beispielsweise in den US-PS 3 020 206, 3 391 060, 3 427 226, 3 433 708, 3 271 267, 3 251 749, 3 281 329, 3 455 786, 3 565 763, 3 594 280, 3 391 061 und 4 119 546 sowie in der DE-OS 2 947 740 beschrieben.

Die von Xanthomonas produzierten hydrophilen Kolloide sind Polysaccharide, die Männose, Glucose, Glucuronsäure, O-Acetyl-Reste und Acetal-verknüpfte Brenztraubensäure enthalten. Sie entstehen als exozelluläres Reaktionsprodukt der genannten Bakterienarten bei deren aeroben Züchtung in wäßrigen Nährlösungen, die neben den üblichen wachstumsfördernden Komponenten insbesondere wasserlösliche Kohlenhydratverbindungen als Kohlenstoffquelle enthalten. In der Praxis wird insbesondere Glucose als Quelle assimilierbaren Kohlenstoffs eingesetzt.

Die Xanthanpolymeren weisen in wäßriger Lösung extreme Verdickungseigenschaften bei gleichzeitiger Thixotropie auf. Als großtechnisches Produkt haben sie heute eine breite Anwendung gefunden. So sind sie wegen ihrer Ungiftigkeit in zahlreichen Anwendungsformen auf dem Nahrungsmittelsektor und in allgemeinen industriellen Anwendungsgebieten zu finden. Besondereres Interesse findet in letzter Zeit ihre Verwendung im Rahmen der sekundären bzw. tertiären Ausbeutung von Öllagerstätten.

Die großtechnische Herstellung des Xanthans unterliegt allerdings — gerade wegen der besonderen Eigenschaften des entstehenden Polymerprodukts — schwerwiegenden Einschränkungen. Das bei der Züchtung in der Fermentermasse anfallende Xanthanprodukt entwickelt bereits hier seine ausgeprägten Verdickungswirkungen, so daß schon bei niedrigen Produktkonzentrationen in der Fermatationsphase Störungen auftreten, die sich beispielsweise in der Gefährdung des Sauerstofftransports und der gleichmäßigen Verteilung des Sauerstoffs im Fermentationsgemisch ausdrücken. Durch den Einsatz hoher Scheerkräfte während der Fermentation kann aufgrund des ausgeprägt thixotropen Verhaltens der entstehenden Xanthan-verdickten wäßrigen Nährlösungen diese Gefährdung des Prozesses noch für einen beschränkten Zeitraum unterdrückt werden. So wird die Fermentation in Reaktionsgefäßen durchgeführt, die intensive Rührwerke, beispielsweise mehrere mehrblättrige Flachschaufelturbinen, enthalten. Unter Einsatz beträchtlicher Rührenergien gelingt es, das ohne Rührung sofort erstarrende Reaktionsgemisch für einen beschränkten Zeitraum noch in so fließfähigem Zustand zu halten, daß beispielsweise der Sauerstofftransport gewährleistet ist. Gleichwohl sind auch unter diesen extremen technischen Bedingungen dem Verfahren enge Grenzen gesetzt. In der Literatur werden maximale Produktausbeuten von etwa 5 Gew.-% Xanthan — berechnet als Trockensubstanz und bezogen auf den Fermatationsansatz — oder auch geringfügig höhere Ausbeuten angegeben. Im praktischen Verfahren sind diese Werte kaum zu erreichen. Eine Ausbeute von 2 bis 3 Gew.-% Xanthan (Trockensubstanz) ist bereits als gutes Ergebnis zu werten und gelingt nur unter Einsatz der zuvor geschilderten stark energieaufwendigen Spezialmaßnahmen.

Die Lehre der vorliegenden Erfindung geht von der Aufgabe aus, die in der Fermatationsstufe nachteilige — beim Endprodukt letztlich aber gerade gewünschte — Verdickungswirkung der Xanthan-Biopolymeren in der Fermetationsstufe so zu mildern bzw. zu beseitigen, daß die Herstellung der Xanthomonas-Polymeren in vielfältiger Weise erleichtert wird. So liegt eine Teilaufgabe der Erfindung in der Senkung der Viskosität der Flüssigphase im Fermenter unter Fermentationsbedingungen bei gleichwohl hohen Produktausbeuten an Xanthan. Die Erfindung will weiterhin ein Züchtungsmedium schaffen, das die Einstellung höherer Feststoffkonzentrationen in der wäßrigen Fermenterphase erlaubt und gleichwohl mit üblichen technischen bzw. apparativen Mitteln verarbeitbar bleibt. Erfindungsgemäß soll die Möglichkeit geschaffen werden, die Xanthangewinnung absatzweise oder auch kontinuierlich mit verringertem Energiebedarf durchzuführen und gegenüber dem Stand der Technik gleiche oder insbesondere auch verbesserte Ausbeuten an gewünschtem Verfahrensprodukt zu ermöglichen.

Zur technischen Lösung dieser Aufgabe wird erfindungsgemäß ein Verfahren zur Herstellung von Xanthomonas-Biopolymeren durch aerobes Züchten von Mikroorganismen der Gattung Xanthomonas in einem wäßrigen Nährmedium vorgeschlagen, daß dadurch gekennzeichnet ist, daß man die Mikroorganismen in einer unter Fermentationsbedingungen stabilen Wasser-in-Öl-Emulsion züchtet. In diesen Emulsionen — die im folgenden der Einfachheit halber als W/O-Emulsionen bezeichnet werden — liegt die wäßrige Fermentationsphase mit ihren darin ablaufenden mikrobiellen Wachstums- bzw. Stoffwechselvorgängen als fein verteilte, disperse Phase in einer homogenen Ölphase vor. In jedem einzelnen Tröpfchen der dispersen wäßrigen Phase kann der mikrobielle Züchtungsschritt ablaufen, eine Versorgung des Einzeltröpfchens mit Sauerstoff ist durch die Belüftung der W/O-Emulsion mit Sauerstoff bzw. einem sauerstoffhaltigen Gas, insbesondere Luft, gewährleistet. Der Viskositätsanstieg im jeweiligen Einzeltröpfchen der wäßrigen Nährlösung macht sich für das im Fermenter vorliegende Gesamtgemisch jedoch nicht oder nicht wesentlich bemerkbar, da die Viskosität der Reaktions-

2

masse im Fermenter weitgehend durch die geschlossene Ölphase bestimmt wird.

Die Lehre der Erfindung knüpft damit in gewissem Sinne an einen Stand der Technik an, wie er aus der sogenannten inversen Emulsionspolymerisation zur Herstellung von Polymerisaten bzw. Mischpolymerisaten wasserlöslicher Monomerer bekannt und beispielsweise in der DE-AS 1 089 173 beschrieben ist. Gleichwohl läßt sich das Verfahren der Erfindung mit diesen Vorschlägen des Standes der Technik nicht vergleichen. Während nach dem Stand der Technik in einer dispersen wäßrigen Phase lediglich die Polymerisationsauslösung zu erfolgen hat, findet im Verfahren der Erfindung in der dispersen wäßrigen Phase ein Prozeß organischen Lebens statt, der den Einfluß bzw. die Gegenwart gasförmigen Sauerstoffs und gegebenenfalls weiterer für das Wachstum bzw. den Stoffwechsel notwendiger Komponenten fordert. Es war nicht vorhersehbar, wie sich die an sich bekannte aerobe Züchtung Xanthan bildender Mikroorganismen in wäßriger Nährlösung verhält, wenn im Sinne der Erfindung das die Mikroorganismen enthaltende wäßrige Substrat als disperse Phase aufgeteilt in einer mit Wasser nicht mischbaren organischen Flüssigphase den an sich bekannten Züchtungsbedingungen unterworfen wird. Überraschenderweise bleiben auch unter diesen Bedingungen die Lebensfähigkeit der Xanthan bildenden Mikroorganismen und damit die Xanthanproduktion erhalten, gleichzeitig tritt jedoch gegenüber dem herkömmlichen Züchtungsverfahren eine substanzielle Senkung der Viskosität in der Fermenter-Flüssigphase ein, die zahlreiche Modifikationen und Verbesserungen gegenüber dem Stand der Technik ermöglicht.

Als organische Flüssigphase sind im erfindungsgemäßen Verfahren grundsätzlich alle mit Wasser nicht mischbaren Flüssigphasen geeignet, die gegenüber den eingesetzten Mikroorganismen nicht toxisch sind, unter Fermentationsbedingungen keiner unerwünschten Veränderung unterliegen — insbesondere also unter Fermentationsbedingungen gegenüber Sauerstoffeinwirkung inert oder im wesentlichen inert sind — und die Ausbildung der W/O-Emulsionen erlauben. Bevorzugt werden organische Flüssigphasen, die im Gleichgewicht mit einer wäßrigen Phase unter Verfahrensbedingungen nur beschränkte Wassermengen aufnehmen. So kann es bevorzugt sein, mit organischen Flüssigphasen zu arbeiten, deren Wassergehalt nicht über 0,5 Gew.-% und insbesondere nicht über 0,1 Gew.-% liegt. Bevorzugt kann es weiterhin sein, solche organischen Flüssigphasen einzusetzen, die auf das entstehende Xanthan-Biopolymere keine oder keine wesentliche solvatisierende und/oder quellende Wirkung ausüben. Die Ölkomponente muß wenigstens bei Fermentationstemperatur als Flüssigphase vorliegen. Bevorzugt liegt ihr Schmelzpunkt nicht über 25°C, insbesondere nicht über 20°C und vorzugsweise nicht höher als 10°C. Weiterhin ist es bevorzugt, daß die Ölphase unter Fermentationsbedingungen nicht oder nur beschränkt flüchtig ist. Wenn hier auch keine zwingende Bedingung vorliegt, so kann doch das Fermentationsverfahren, das ja beispielsweise mit einem durch das Fermentationsgemisch durchgeleiteten Luftstrom arbeitet, bedeutend vereinfacht werden. Auf der anderen Seite ist zu berücksichtigen, daß in bestimmten Ausführungsformen der Erfindung bei der Aufarbeitung des Verfahrensproduktes die teilweise oder vollständige Abtrennung der Ölphase wünschenswert ist, so daß hier auf die jeweiligen Gesamtbedingungen zugeschnitten im Einzelfall optimale Verhältnisse eingestellt werden können.

Durch einfache Vorversuche läßt sich jeweils ermitteln, ob die Grundbedingung der toxikologischen Verträglichkeit der jeweils ausgewählten Ölphase mit dem Mikroorganismenwachstum gewährleistet ist. Tatsächlich ist offenbar in breitem Rahmen organischer Flüssigphasen diese Voraussetzung gegeben.

Bei der Ölphase kann es sich weiterhin bevorzugt um beliebige hydrophobe Flüssigkeiten handeln, die von der entstehenden polymeren wäßrigen Phase hinreichend leicht getrennt werden können. Geeignet sind unsubstituierte und/oder substituierte flüssige Kohlenwasserstoffverbindungen. Diese Bestimmung umfaßt sowohl aliphatische als auch aromatische Verbindungen. Aliphatische Verbindungen können geradkettig, verzweigt oder cyclisch sein. Es kann sich dabei um bestimmte einzelne Kohlenwasserstoffverbindungen oder um Mischungen verschiedener Verbindungen handeln. Geeignet sind beispielsweise Kohlenwasserstofffraktionen des Bereichs der Mineralöle, der Kerosine oder Naphthas, aber auch organische Kohlenwasserstoffe wie Benzol, Xylol oder Toluol. Besonders geeignet kann eine Ölphase auf Basis verzweigter Kohlenwasserstoffverbindungen, beispielsweise auf Isoparaffinbasis aufgebaut sein. Es sind aber nicht nur unsubstituierte, gegebenenfalls ungesättigte, insbesondere olefinisch ungesättigte Kohlenwasserstoffverbindungen geeignet, auch hinreichend wasserunlösliche Alkohole mit insbesondere 8 bis 20, vorzugsweise 8 bis 12 C-Atomen, pflanzliche Öle, Esteralkohole, Polyoläther, oder andere Heteroatome enthaltende Verbindungen, beispielsweise Siliconöle sind geeignet. Ein unter dem Handelsnamen »Isopar M« vertriebenes Isoparaffingemisch bzw. ein partielles Neutralisationsprodukt aus wasserunlöslichen Isoparaffinsäuren, die mit hydroxibenzyldialiphatischen Aminen teilneutralisiert worden sind und im einzelnen in der US-PS 2 262 720 beschrieben sind, hat sich als brauchbar erwiesen.

Das wäßrige Fermentationsmedium kann unter den in der Literatur für die Xanthanproduktion beschriebenen ausgewählt werden. Geeignete wäßrige Fermentationsmedien sind beispielsweise in der US-PS 4 119 546 und in der DE-OS 2 947 740 beschrieben. Typische wäßrige Nährlösungen enthalten beispielsweise eine Quelle für organischen Stickstoff wie Maisquellwasser und/oder Sojamehl, Phosphatsalze wie Dialkalihydrogenphosphat und/oder Diammoniumhydrogenphosphat neben geeigneten Spurenelementen, insbesondere Magnesium und gegebenenfalls Mangan, Eisen und Calci-

3

um bei einem pH-Wert oberhalb 6, vorzugsweise oberhalb von 6,5 bis etwa 7. Die Nährlösung enthält zusätzlich ein für die Xanthanproduktion geeignetes Kohlenhydrat zweckmäßigerweise im wäßrigen Nährmedium gelöst. Geeignete Kohlenhydrate sind z. B. Glucose, Saccharose, Maltose, Fructose, Lactose, bearbeitete, invertierte Zuckerrüben-Melassen, Invertzucker, filtrierte verdünnte Qualitätsstärke oder Gemische dieser Kohlenhydrate. Glucose ist die bevorzugte Quelle des assimilierbaren Kohlenstoffs. Die Konzentration der assimilierbaren Kohlenhydratverbindung liegt üblicherweise im Bereich von 2 bis 5 Gew.-%, bezogen auf die wäßrige Phase. Die Verwendung höherer Glucosekonzentrationen kann zur Ansammlung toxischer saurer Nebenprodukte und damit zu einer Hemmung des Xanthomonaswachstums führen, die sogar eine vorzeitige Beendigung der Fermentation auslösen kann. Möglich ist jedoch die Zugabe der assimilierbaren Kohlenhydratverbindung absatzweise oder kontinuierlich während des Fermentationsverlaufs, so daß letztlich beträchtlich höhere Glucosekonzentrationen umgesetzt werden können. Die Inkubationstemperatur liegt zweckmäßigerweise im Bereich von etwa 30° C, beispielsweise bei 30 ± 5° C kann bis zu einem Zeitraum von etwa 100 Stunden oder auch länger durchgeführt werden. Ein in der Praxis üblicherweise eingesetztes Verfahren zur Herstellung von Xanthan und die dabei eingesetzten Reaktionshilfsmittel, insbesondere Nährlösungen und Inkubationsbedingungen sind beispielsweise in der US-PS 3 236 831 beschrieben.

Die Auswahl und Abstimmung der geeigneten Ölphasen und Nährlösungen wird einerseits durch die erfindungsgemäß gewünschte optimale Erleichterung der Züchtungsstufe bestimmt, andererseits kann jedoch auch die beabsichtigte Verwendung des Xanthanproduktes in die Auswahl insbesondere der Ölphase eingehen. Soll beispielsweise das Xanthanprodukt auf dem Gebiet der sekundären bzw. tertiären Ausbeutung von Öllagerstätten eingesetzt werden, dann können unbedenklich Kohlenwasserstoffverbindungen als Ölphase Verwendung finden, die ernährungsphysiologisch weniger geeignet sein könnten. Ist daran gedacht, das Xanthanprodukt dem Nahrungsmittelsektor zuzuführen, so kann es bevorzugt sein, mit einer Ölphase zu arbeiten, die aus sich heraus keine ernährungsphysiologischen Bedenken auslöst.

Zur Ausbildung der W/O-Emulsionen kann es bevorzugt sein, Emulgierhilfsmittel einzusetzen. Emulgiermittel vom Wasser-in-Öl-Typ (W/O-Emulgatoren) sind in der chemisch-technischen Praxis vielfach beschrieben. Die Emulgatoren lösen sich vorzugsweise in der Ölphase und führen bei der Verarbeitung mit einer wäßrigen Phase zur Ausbildung des angestrebten W/O-Typs. Geeignete Verbindungen sind beispielsweise Sorbitanmonooleat, Sorbitanmonostearat, Hexadecylnatriumphthalat, Cetylstearylnatriumphthalat und weitere Verbindungen, wie sie in der DE-AS 1 089 173 genannt sind. Geeignet sind weiterhin W/O-Emulgatorsysteme, wie sie beispielsweise von der Firma Atlas Chemie GmbH unter den Handelsbezeichnungen »ARLACEL- oder SPAN-Produkte« vertrieben werden, wobei es sich um Verbindungen aus der Gruppe der Oleate mit einem geringen Anteil an »TWEEN«-Emulgatoren handelt. Weitere geeignete W/O-Emulgatorsysteme sind von der genannten Firma Atlas Chemie GmbH beschrieben und werden in der Literatur referiert als »Atlas-HLB-Systeme«, vergleiche hierzu beispielsweise US-PS 3 996 180 und das einschlägige Prospektmaterial der genannten Firma. Zahlreiche W/O-Emulgatoren sind weiterhin beschrieben in Detergents and Emulsifier's Annual, John W. McCutcheon, Inc. Morristown N. J.

Die Auswahl der jeweils geeigneten Emulgierhilfsmittel kann wiederum durch die angestrebte Verfahrensführung und/oder die angestrebte Beschaffenheit des Verfahrensendproduktes bestimmt sein. So kann es im Rahmen des erfindungsgemäßen Handelns liegen, soweit stabilisierte W/O-Emulsionen herzustellen, daß nach Verfahrensabschluß eine vollständige Trennung der Ölphase von der viskosen wäßrigen Phase nur mit Schwierigkeiten verbunden wäre. Dieser Fall kann beispielsweise bei dem beabsichtigten Einsatz des Xanthans bei der Ausbeutung von Öllagerstätten gegeben sein. Auf der anderen Seite kann es beispielsweise ein im folgenden noch zu schildernde Verfahrensmodifikation wünschenswert machen, daß eine relativ einfache Trennung zwischen Ölphase und wäßriger Phase eintritt. Dieser Fall kann beispielsweise gegeben sein, wenn entweder das Reaktionsprodukt als ganzes oder ein aus dem Fermenter abgezogener Teilstrom der W/O-Emulsion in beide Phasen getrennt und gegebenenfalls ein Anteil des abgetrennten Produktes wieder in den Fermenter zurückgeleitet werden soll. Für diesen zuletzt genannten Fall kann sich die Erfindung die Tatsache zunutze machen, daß auch im erfindungsgemäßen Verfahren in der bevorzugten Ausführungsform im Fermenter unter ständiger Vermischung des Reaktionsgutes gearbeitet wird, so daß also unter den Arbeitsbedingungen der Fermentation die Einstellung der W/O-Emulsion gewährleistet ist, bei Ruhigstellung des Reaktionsgemisches jedoch eine hinreichend leichte Trennung der Phasen ermöglicht wird. Je nach den Besonderheiten des Einzelfalles wird die Auswahl der W/O-Emulgatoren vorgenommen.

Gegebenenfalls können im Verfahren der Erfindung weitere Hilfsmittel im Fermentationsansatz mitverwendet werden. Ein Beispiel hierfür sind Dispergierhilfsmittel.

Bevorzugte Mischungsverhältnisse von Ölphase zu der wäßrigen Nährlösung liegen im Bereich von 15 bis 90 Gewichtsanteile Ölphase auf 85 bis 10 Gewichtsanteile wäßriger Nährlösung bei Reaktionsbeginn. Insbesondere macht die Ölphase wenigstens 20 Gew.-% des W/O-Gemisches aus, wobei Ölmengen im Bereich von 20 bis 60 Gew.-% besonders bevorzugt sein können. Als im allgemeinen zweckmäßig haben sich Ölmengen im Bereich von etwa 25 bis 50 Gew.-% — bezogen auf die W/O-Mischung — erwiesen.

Werden W/O-Emulgatoren mitverwendet, so liegt ihre Menge häufig im Bereich von etwa 0,1 bis 10

4

Gew.-%, bezogen auf das Gesamtgemisch von W/O-Phase. Der bevorzugte Bereich für den Emulgatorgehalt liegt hier bei etwa 1 bis 5 Gew.-%. Es kann dabei weiterhin bevorzugt sein, daß der Emulgatorgehalt — bezogen auf die Ölphase — 3 bis 20 Gew.-%, insbesondere etwa 5 bis 15 Gew.-%, ausmacht. Die im Einzelfall gewählte Menge des Emulgierhilfsmittels kann dabei — in Abstimmung mit seinen durch die chemische Konstitution bestimmten Emulgiereigenschaften — bestimmt werden durch die zuvor geschilderte Absicht, stabile oder weniger stabile W/O-Emulsionen herzustellen.

Geeignete Xanthomonasorganismen, die exozelluläre Heteropolysaccharide bilden können, leiten sich beispielsweise aus den folgenden Xanthomonas-Species ab: Xanthomonas campestris, Xanthomonas phaseoli, Xanthomonas malvacearum, Xanthomonas translucens, Xanthomonas carotae, Xanthomonas hederae, Xanthomonas papavericola, Xanthomonas begoniae, Xanthomonas incanae, Xanthomonas vasculorum und Xanthomonas vesicatoria. Geeignet sind insbesondere Stämme von Xanthomonas campestris aber auch solche von Xanthomonas fragaria, Xanthomonas gummisudans, Xanthomonas manihotis und Xanthomonas vasculorum.

Ein sauerstoffhaltiges Gas, insbesondere Luft, wird in den Produktionsfermenter mit herkömmlichen Mitteln eingeführt. Der Sauerstoffbedarf für die Fermentation kann den Gegebenheiten des Fermentationsverfahrens und des Sauerstofftransports angepaßt werden, um die Ansammlung sauer toxischer Nebenprodukte herabzusetzen.

Gegenüber dem herkömmlichen Verfahren der Xanthanproduktion bringt die erfindungsgemäße Konzeption des Arbeitens in W/O-Emulsionen die Möglichkeit zu vielgestaltigen Abwandlungen unter Einstellung verbesserter Verfahrensbedingungen bzw. Ergebnisse. Im einzelnen gilt hier das folgende

Die Anzüchtung der im großtechnischen Verfahren eingesetzten Mikroorganismen kann mehrstufig in Nährmedien unterschiedlicher Zusammensetzung erfolgen, so wie es beispielsweise in der DE-OS 2 947 740 beschrieben und auf dem hier betroffenen Arbeitsgebiet ganz allgemein bekannt ist. Die so vorbereiteten Mikroorganismen werden dann in die für die Produktion vorgesehenen Nährlösungen gegeben und hier gezüchtet.

In einer Ausführungsform der Erfindung kann schon in dieser frühen Stufe die W/O-Emulsion ausgebildet werden, so daß — dargestellt anhand einer absatzweisen Verfahrensführung — bereits die Stufe der Vermehrung der Mikroorganismen in der erfindungsgemäßen Emulsion erfolgt. Damit überlappend und anschließend bildet sich als exozelluläres Stoffwechselprodukt der Mikroorganismenzüchtung das Xanthan-Heteropolysaccharid.

In einer erfindungsgemäß bevorzugten Ausführungsform kann es allerdings zweckmäßig sein, die Xanthan bildenden Mikroorganismen zunächst im ölfreien wäßrigen Nährmedium anzuzüchten, dabei die Vermehrung der Mikroorganismen in Abwesenheit der Ölphase zu einem nicht unbeträchtlichen Anteil voranzutreiben und erst dann die derart vorgezüchtete wäßrige Fermentationslösung in die W/O-Emulsion zu überführen. In dieser Ausführungsform wird zweckmäßigerweise so vorgegangen, daß die Emulgierung bzw. Dispergierung der wäßrigen Phase in der Ölphase zu einem Zeitpunkt erfolgt, bei dem die sichere Verarbeitbarkeit der durch Xanthanbildung erstarrenden wäßrigen Phase noch gewährleistet ist. So kann es in dieser Ausführungsform zweckmäßig sein, daß man die ölfreie Anzüchtung in der wäßrigen Phase bis zu Ausbeuten von wenigstens 0,1 Gew.-% Biopolymerem — vorzugsweise bis zu Ausbeuten von wenigstens 0,5 Gew.-% Biopolymerem — durchführt, bevor die Ausbildung der W/O-Emulsion vorgenommen wird. Die hier angegebenen Zahlenwerte für Gew.-% beziehen sich dabei jeweils auf die Trockensubstanz des Biopolymeren, bezogen auf das Gewicht des wäßrigen Fermentationsansatzes. Im einstufigen Verfahren ist es dabei möglich, die Vermehrungsstufe des Mikroorganismus zu wenigstens 30%, gegebenenfalls zu wenigstens 50% in der wäßrigen Nährlösung ablaufen zu lassen, bevor die Überführung in die W/O-Emulsion erfolgt. Durch einfache Vorversuche kann ein jeweils optimaler Zeitraum bestimmt werden, innerhalb dessen die sichere Überführung der wäßrigen Phase in die disperse Aufteilung in der geschlossenen Ölphase unter den erfindungsgemäßen Verfahrensbedingungen noch möglich ist.

Bezogen auf Trockensubstanz des gebildeten Xanthans in der wäßrigen Nährlösung ist das selbst dann noch der Fall, wenn hier Produktausbeuten im Bereich von 1,0 bis 1,5 Gew.-% oder sogar darüber bis zu 2 Gew.-% vorliegen.

Zur Ausbildung der W/O-Emulsion werden die angezüchtete Nährlösung und die Ölphase — in der zweckmäßigerweise zuvor der gegebenenfalls mitverwendete Emulgator gelöst worden ist — miteinander vermischt und das Gemisch hinreichend intensiv mechanisch bewegt bzw. durchgearbeitet. In der bevorzugten Ausführungsform des erfindungsgemäßen Verfahrens erfolgt die Züchtung auch innerhalb der W/O-Emulsion in Fermentern, die mit intensiv arbeitenden Mischelementen ausgerüstet sind. Die intensive Durchmischungswirkung solcher Rührelemente kann erfindungsgemäß dazu ausgenutzt werden, im Fermenter den Zustand der W/O-Emulsion aufrechtzuerhalten und sicherzustellen, und zwar selbst in solchen Fällen, in denen bei Ruhigstellung des Fermenterinhalts eine relativ einfache Phasenentmischung eintritt.

Der bewegte Fermenterinhalt wird in konventioneller Weise mit Sauerstoff bzw. mit einem sauerstoffhaltigen Gas, insbesondere Luft, begast. Der Sauerstofftransfer erfolgt über die Ölphase an jedes einzelne in disperser Phase emulgierte Teilchen der wäßrigen, Mikroorganismen enthaltenden Nährlösung und ist dementsprechend auch in späten Stadien des Verfahrens weitaus weniger gestört als bei der bisher üblichen Verfahrenstechnik.

—

5

# 0 058 364

Erforderlichenfalls können wachstumsfördernde Bestandteile der Nährlösung absatzweise oder kontinuierlich nachgeliefert werden. So ist es etwa möglich, die assimilierbaren Kohlenstoff enthaltenden Verbindungen, wie Glucose, nach und nach dem Reaktionsgemisch zuzusetzen. Eine gleichmäßige Verteilung kann dadurch sichergestellt werden, daß diese Nährstoffe dem intensiv durchgearbeiteten Fermenter unmittelbar zugegeben werden. Entsprechendes gilt für andere Bestandteile der wäßrigen Nährlösung beispielsweise für Spurenelemente oder auch für die Zugabe von sonstigen benötigten Reaktanten beispielsweise Basen zur Regulierung des pH-Werts der dispersen wäßrigen Phase.

Die Züchtung wird dann so lange fortgeführt, bis die gewünschte Xanthanausbeute eingestellt ist bzw. die Xanthanbildung absinkt oder gar zum Stillstand kommt. Im absatzweisen Verfahren wird dann — je nach dem beabsichtigten Verwendungszweck des Xanthanprodukts — in der Regel eine Trennung zwischen Ölphase und xanthanhaltiger disperser Phase erfolgen, woraufhin das Xanthan in konventioneller Weise aus der wäßrigen Phase abgetrennt und gereinigt werden kann. Zum Brechen der W/O-Emulsionen können die in der chemischen Verfahrenspraxis üblichen Maßnahmen einge- setzt werden, vergleiche hierzu beispielsweise Ullmann Enzyklopädie der technischen Chemie 1975, Band IV, Seite 453 und ff., sowie Houben—Weyl, Methoden der Organischen Chemie 1958, Band I/1, Seiten 219/220. Die Emulsionstrennung kann dementsprechend durch Zugabe emulsionsbrechender Substanzen, gegebenenfalls durch Einwirkung mechanischer Kräfte wie Schütteln, Schlagen oder Druckeinwirkung, durch Temperaturerhöhung, durch Verdünnen oder Eindampfen der äußeren Phase und andere bekannte Maßnahmen erfolgen. Insbesondere die Zerstörung von Emulsionen durch Zusatz geeigneter chemischer Stoffe, der sogenannten Desmulgatoren, ist in der Verfahrenstechnik bekannt. Der Handel hat eine Vielzahl von Emulsionsspaltern entwickelt, vergleiche hierzu beispielsweise Houben—Weyl aaO. Übliche Reinigungsschritte, beispielsweise das Auswaschen der xanthanhaltigen wäßrigen Phase mit geeigneten Lösungsmitteln, können sich anschließen.

In einer besonderen Ausführungsform des erfindungsgemäßen Verfahrens kann ein Teilstrom des Fermenterinhaltes absatzweise oder kontinuierlich abgezweigt, aufgearbeitet und gewünschtenfalls wenigstens anteilsweise wieder zurückgegeben werden. Über den abgezweigten Teilstrom kann beispielsweise die Zugabe von Reaktionshilfsmitteln oder aber auch die Zugabe frischer Mikroorganismenanteile erfolgen. Gleichzeitig kann über einen solchen abgezweigten Teilstrom eine absatzweise oder kontinuierliche Ausschleusung von Verfahrensprodukt aus dem Fermenter erfolgen. Für die Erfindung eröffnet sich hiermit die Möglichkeit der kontinuierlichen Verfahrensführung, wobei insbesondere durch regelmäßige Kontrolle der Xanthan enthaltenden wäßrigen Phase die Steuerung des Verfahrensablaufs in gewünschter Richtung möglich ist. Gegenüber dem in der Praxis bisher üblichen Verfahrenstyp des absatzweise arbeitenden einstufigen Verfahrens erschließen sich hier neue technisch fortschrittliche Möglichkeiten.

Die Isolierung des Xanthans aus der wäßrigen Phase erfolgt in an sich bekannter Weise beispielsweise durch Fällung und Trocknung. Zunächst kann die xanthanhaltige Phase hinreichend — zum Beispiel auf Temperaturen über $100°C$ — erhitzt und sofort wieder abgekühlt werden, um die vorhandenen Mikroorganismen abzutöten und gegebenenfalls die Viskosität des Xanthans zu verbessern. Xanthan wird dann durch Fällung beispielsweise mit Alkoholen, anschließendes Filtrieren und Trocknen gewonnen. Waschstufen zur Reinigung des Xanthanprodukts können in an sich bekannter Weise eingeschlossen sein.

Beispiel 1

Xanthomonas campestris NRRL-B-1459-A wird im 7-l-Fermenter (5 l Füllvolumen) in dem folgenden Nährmedium bei $27°C$ aerob angezüchtet:

2,80 Gew.-% Glucose (getrennt sterilisiert)
0,60 Gew.-% Sojamehl
1,00 Gew.-% Maisquellwasser
0,02 Gew.-% $MgSO_4 \times 7 H_2O$
0,08 Gew.-% $(NH_4)_2HPO_4$
0,09 Gew.-% $Na_2HPO_4$

Der pH-Wert der Nährlösung beträgt 6,8.

Nach 24stündiger Inkubation erfolgt die Zugabe von weiteren 2,8 Gew.-% Glucose. In einem ersten Kontrollansatz wird unter intensivem Durchmischen des Fermenteransatzes und gleichmäßiger Belüftung ohne Zusatz einer Ölphase die Fermentation bis zu einer Inkubationszeit von insgesamt 64 Stunden fortgeführt. Die Viskosität des Fermenterinhalts und die Xanthanausbeute werden nach Ablauf von 16, 22, 40, 47 und 64 Stunden Inkubationszeit bestimmt. Die dabei ermittelten Zahlenwerte sind in der folgenden Tabelle zusammengefaßt.

In einem 2. Versuch wird zunächst in völlig gleicher Weise gearbeitet. Auch hier erfolgt nach 24stündiger Inkubation die Zugabe von weiteren 2,8 Gew.-% Glucose. Nach 40stündiger Inkubation

6

wird jedoch eine Sorbitanmonooleat (»Span 80«) enthaltende Ölphase zugegeben und hierdurch eine W/O-Emulsion ausgebildet. Als Ölphase wurde »Isopar M« (Warenbezeichnung der Firma ESSO Chemie für ein Isoparaffingemisch des Siedebereichs von etwa 200 bis 250°C und einem Aromatengehalt von 0,3 Gew.-%) eingesetzt. Die Menge der Ölphase beträgt 30 Gew.-%, bezogen auf das Gesamtgemisch. Der Emulgatorgehalt beträgt 3 Gew.-%.

Auch bei diesem Ansatz werden die Viskosität und die Xanthanausbeute nach jeweils 16, 22, 40, 47 und 64 Stunden Inkubationszeit bestimmt. In der folgenden Tabelle sind die dabei erhaltenen Verfahrensergebnisse gegenübergestellt:

Tabelle

| Fermentationsbedingungen | Inkubations-zeit (Std.) | Viskosität[1] (c.P.) | Xanthan-ausbeute[2] (g/l) |
|---|---|---|---|
| Kontrollansatz | 16 | 650 | — |
| | 22 | 1 700 | 8,3 |
| | 40 | 7 300 | 16,0 |
| | 47 | 9 200 | 19,0 |
| | 64 | 10 000 | 18,2 |
| W/O-Ansatz unter Zugabe von 3% Span 80 plus 30% Isopar M | 16 | 850 | — |
| | 22 | 2 400 | 12,3 |
| | 40 | 9 500 | 17,9 |
| | — | 500[3] | — |
| | 47 | 750 | 20,0 |
| | 64 | 500 | 25,5 |

[1]  Brookfield-Viskosimeter, Spindel 2—5, Geschwindigkeit 20
[2]  Trockengewicht nach Präzipitierung
[3]  nach Zugabe von Span 80 und Isopar M

Der Kontrollansatz zeigt, daß unter den gewählten Verfahrensbedingungen die Xanthanproduktion nach einer Inkubationszeit von 47 Stunden praktisch zum Stillstand kommt. Demgegenüber zeigt der Ansatz gemäß der Erfindung im Zeitraum der Inkubation von 47 bis 67 Stunden einen gegenüber der Anfangsphase der Züchtung (Verfahrenszeitraum von 22 bis 40 Stunden) gleichbleibend kräftigen Anstieg der Xanthanproduktion. Durch den Zusatz der Ölphase nach 40stündiger Inkubation sinkt die Viskosität des Fermenterinhalts im erfindungsgemäßen Verfahren auf den Anfangswert und steigt trotz ungebrochenen Xanthanwachstums nicht weiter an.

In einem 3. Versuch wird zunächst in völlig gleicher Weise gearbeitet. Auch hier erfolgt nach 24stündiger Inkubation die Zugabe von weiteren 2,8 Gew.-% Glucose und nach 40stündiger Inkubation die Zugabe von »Span 80« unter Einsatz von 30 Gew.-% »Isopar M« als Ölphase. Die Menge an »Span 80« beträgt diesmal jedoch 1,5 Gew.-%.

Es wird eine Xanthanausbeute von 25 g/l erhalten.

Beispiel 2

Xanthomonas campestris NRRL-B 1459-A wird in gleicher Weise wie in Beispiel 1 gezüchtet, jedoch wird diesmal folgende organische Phase verwendet:

**0 058 364**

2,2 Gew.-% Glycerinstearat (Atmos 300)
4,4 Gew.-% Polyoxyethylenoleyläther (BRIJ 92)
43,4 Gew.-% Isotridecylstearat (Rilanit ITS).

Es wird eine Xanthanausbeute von 24,5 g/l erhalten.

## Patentansprüche

1. Verfahren zur Herstellung von Xanthomonas-Biopolymeren durch aerobes Züchten von Mikroorganismen der Gattung Xanthomonas in einem wäßrigen Nährmedium, dadurch gekennzeichnet, daß man die Mikroorganismen in einer unter Fermentationsbedingungen stabilen Wasser-in-Öl-Emulsion (W/O-Emulsion) züchtet.

2. Verfahren nach Anspruch 1, dadurch gekennzeichnet, daß man den Mikroorganismus zunächst im ölfreien, wäßrigen Nährmedium anzüchtet, dann unter Zusatz der Ölphase eine W/O-Emulsion ausbildet und unter Aufrechterhaltung der W/O-Emulsion die Fermentation weiterführt.

3. Verfahren nach Ansprüchen 1 und 2, dadurch gekennzeichnet, daß man die ölfreie Anzüchtung bis zu Ausbeuten von wenigstens 0,1 Gew.-%, vorzugsweise von wenigstens 0,5 Gew.-% Biopolymeren (berechnet als Trockensubstanz, bezogen auf das Gewicht des wäßrigen Fermentationsansatzes) durchführt, bevor die Ausbildung der W/O-Emulsion vorgenommen wird.

4. Verfahren nach Ansprüchen 1 bis 3, dadurch gekennzeichnet, daß man zur Ausbildung der W/O-Emulsion Wasser-in-Öl-Emulgatoren und Ölphase dem angezüchteten wäßrigen Reaktionsansatz zugibt und das Gemisch mechanisch bewegt, wobei vorzugsweise zunächst der W/O-Emulgator in der Ölphase gelöst und diese Lösung dann mit der wäßrigen Phase vermischt wird.

5. Verfahren nach Ansprüchen 1 bis 4, dadurch gekennzeichnet, daß man die Ölphase in Mengen von wenigstens 15 Gew.-%, vorzugsweise von wenigstens 25 Gew.-% — jeweils bezogen auf das gesamte Fermentationsgemisch — einsetzt und insbesondere mit Ölmengen von nicht mehr als 90 Gew.-% arbeitet.

6. Verfahren nach Ansprüchen 1 bis 5, dadurch gekennzeichnet, daß man mit W/O-Emulsionen arbeitet, in denen die Ölmenge im Bereich von 25 bis 50 Gew.-% — bezogen auf die W/O-Emulsion — liegt.

7. Verfahren nach Ansprüchen 1 bis 6, dadurch gekennzeichnet, daß man als Ölphase mit Wasser nicht mischbare, unter Fermetationsbedingungen flüssige und den eingesetzten Mikroorganismus gegenüber nicht-toxische organische Lösungsmittel einsetzt.

8. Verfahren nach Ansprüchen 1 bis 7, dadurch gekennzeichnet, daß man die W/O-Emulgatoren in Mengen von etwa 0,1 bis 10 Gew.-%, vorzugsweise von etwa 1 bis 5 Gew.-% — jeweils bezogen auf das Fermetationsgesamtgemisch — einsetzt, wobei Mengen von etwa 5 bis 15 Gew.-% W/O-Emulgator — bezogen auf die Ölphase — bevorzugt sein können.

9. Verfahren nach Ansprüchen 1 bis 3, dadurch gekennzeichnet, daß man mit einem Stamm von Xanthomonas campestris arbeitet.

10. Verfahren nach Ansprüchen 1 bis 9, dadurch gekennzeichnet, daß man in Gegenwart assimilierbarer Kohlenhydratverbindungen arbeitet, wobei vorzugsweise Glucose, Saccharose, Maltose, Fructose, Lactose, Invertzucker und/oder solche Komponenten enthaltende Naturstoffe eingesetzt werden.

11. Verfahren nach Ansprüchen 1 bis 10, dadurch gekennzeichnet, daß man die Fermentation bei fortlaufendem Vermischen des Fermenterinhalts vorzugsweise unter intensivem Rühren der W/O-Emulsion durchführt.

## Claims

1. A process for the production of xanthomonas biopolymers by the aerobic culture of microorganisms of the genus xanthomonas in an aqueous nutrient medium, characterized in that the microorganisms are cultured in a water-in-oil emulsion (W/O-emulsion) which is stable under fermentation conditions.

2. A process as claimed in Claim 1, characterized in that the microorganism is first cultured in the oil-free aqueous nutrient medium, after which a W/O-emulsion is formed by addition of the oil phase and fermentation is continued with the W/O-emulsion intact.

3. A process as claimed in Claims 1 and 2, characterized in that the oil-free culture is carried out up to yields of at least 0.1% by weight and preferably up to yields of at least 0.5% by weight of biopolymer (expressed as dry matter, based on the weight of the aquous fermentation mixture) before the W/O-emulsion is formed.

4. A process as claimed in Claims 1 to 3, characterized in that, to form the W/O-emulsion, water-in-oil emulsifiers and oil phase are added to the aqueous initial culture and the mixture is mechanically agitated, the W/O-emulsifier preferably being dissolved in the oil phase and the resulting solution

8

0 058 364

being mixed with the aqueous phase.

5. A process as claimed in Claims 1 to 4, characterized in that the oil phase is used in quantities of at least 15% by weight and preferably in quantities of at least 25% by weight, based on the fermentation mixture as a whole, and more particularly in quantities of no more than 90% by weight.

6. A process as claimed in Claims 1 to 5, characterized in that the W/O-emulsions used contain from 25 to 50% by weight of oil, based on the W/O-emulsion.

7. A process as claimed in Claims 1 to 6, characterized in that water-immiscible organic solvents which are liquid under fermentation conditions and which are non-toxic to the microorganism used are used as the oil phase.

8. A process as claimed in Claims 1 to 7, characterized in that the W/O-emulsifiers are used in quantities of from about 0.1 to 10% by weight and preferably in quantities of from about 1 to 5% by weight, based on the fermentation mixture, and advantageously in quantities of from about 5 to 15% by weight, based on the oil phase.

9. A process as claimed in Claims 1 to 8, characterized in that a strain of Xanthomonas campestris is used.

10. A process as claimed in Claims 1 to 9, characterized in that it is carried out in the presence of assimilable carbohydrate compounds, preferably glucose, sucrose, maltose, fructose, lactose, invert sugar, and/or natural substances containing such compounds.

11. A process as claimed in Claims 1 to 10, characterized in that fermentation is carried out with continuous mixing of the fermenter contents and preferably with intensive stirring of the W/O-emulsion.

## Revendications

1. Procédé pour la fabrication de biopolymères de Xanthomonas par culture aérobie de micro-organismes de l'espèce Xanthomonas, dans un milieu nutritif aqueux, procédé caractérisé en ce qu'on cultive les micro-organismes dans une émulsion eau dans huile (émulsion W/O) stable dans les conditions de fermentation.

2. Procédé suivant la revendications 1, caractérisé en ce que l'on cultive les micro-organismes d'abord dans un milieu nutritif aqueux exempt d'huile, on forme ensuite une émulsion W/O en ajoutant la phase huile et on poursuit la fermentation en maintenant l'émulsion W/O.

3. Procédé suivant les revendications 1 et 2, caractérisé en ce que l'on effectue la culture exempte d'huile jusqu'à arriver à un rendement d'au moins 0,1% en poids, notamment d'au moins 0,5% en poids de biopolymère (calculé en substance sèche par rapport au poids de la charge de fermentation aqueuse), avant de former l'émulsion W/O.

4. Procédé suivant les revendications 1 à 3, caractérisé en ce que l'on ajoute à la charge réactionnelle aqueuse cultivée, en vue de la formation de l'émulsion W/O, des émulsionnants eau-dans-huile et une phase huile, on agite le mélange mécaniquement, notamment en dissolvant d'abord l'émulsionnant W/O dans la phase huile, et on mélange ensuite cette solution avec la phase aqueuse.

5. Procédé suivant les revendications 1 à 4, caractérisé en ce que l'on met en œuvre la phase aqueuse dans une proportion d'au moins 15% en poids, ou mieux d'au moins 25% en poids, toujours calculé sur la totalité du mélange de fermentation, et on opère en particulier avec des proportions d'huile qui ne dépassent pas 90% en poids.

6. Procédé suivant l'une des revendications 1 à 5, caractérisé en ce qu'on opère avec des émulsions W/O, dans lesquelles la proportion d'huile est de l'ordre de 25 à 50% en poids, calculé sur l'émulsion W/O.

7. Procédé suivant l'une des revendications 1 à 6, caractérisé en ce que l'on met en œuvre, comme phase huile non miscible à l'eau, des solvants organiques liquides dans les conditions de la fermentation et non toxiques vis-à-vis des micro-organismes mis en œuvre.

8. Procédé suivant les revendications 1 à 7, caractérisé en ce que l'on met en œuvre l'émulsionnant W/O en proportions de 0,1 à 10% en poids, de préférence de 1 à 5% en poids, toujours calculé sur la totalité du mélange de fermentation, bien que l'on puisse recommander des proportions de 5 à 15% en poids d'émulsionnant W/O calculé sur la phase huile.

9. Procédé suivant l'une des revendications 1 à 8, caractérisé en ce que l'on opère avec une souche de Xanthomonas campestris.

10. Procédé suivant les revendications 1 à 9, caractérisé en ce que l'on opère en présence de composés hydrates de carbone assimilables, en mettant en œuvre de préférence les glucose, saccharose, maltose, fructose, lactose, sucre interverti, et/ou des substances naturelles contenant ces composants.

11. Procédé suivant les revendications 1 à 10, caractérisé en ce que l'on effectue la fermentation en poursuivant le travail de mélange du contenu de la cuve de fermentation, notamment en agitant énergiquement l'émulsion W/O.

9